(19) Europäisches Patentamt
European Patent Office
Office européen des brevets

(11) Veröffentlichungsnummer: **0 119 476**
**A2**

# (12) EUROPÄISCHE PATENTANMELDUNG

(21) Anmeldenummer: **84101610.8**

(22) Anmeldetag: **16.02.84**

(51) Int. Cl.³: **C 12 N 5/00**, C 12 N 15/00, C 12 P 21/00, C 07 C 103/52, B 01 D 15/08, G 01 N 33/54 // A61K45/02

(30) Priorität: **22.02.83 DE 3306060**

(43) Veröffentlichungstag der Anmeldung: **26.09.84 Patentblatt 84/39**

(84) Benannte Vertragsstaaten: **AT BE CH DE FR GB IT LI LU NL SE**

(71) Anmelder: **Boehringer Ingelheim International G.m.b.H, D-6507 Ingelheim am Rhein (DE)**

(72) Erfinder: **Adolf, Günther, Dr. Mag., Johannagasse 20/7, A-1120 Wien (AT)**
Erfinder: **Bodo, Gerhard, Prof. Dr., Belghofergasse 27, A-1120 Wien (AT)**
Erfinder: **Swetly, Peter, Dr., Hietzinger Hauptstrasse 40 B, A-1130 Wien (AT)**

(54) **Neue Immunglobulin-produzierende Hybridzellinien, deren Verwendung und Verfahren zu deren Herstellung.**

(57) Die Erfindung betrifft neue Immunglobulin-produzierende Hybridzellinien, welche durch Zellfusion von Myelomzellen mit gegen Humaninterferon des Typs α immunisierten Mäuse-Milzzellen nach bekannten Methoden hergestellt werden, die beim Wachstum dieser Zellen gebildeten Antikörper und deren Verwendung bei der Reinigung, der Diagnose und dem Nachweis von Humaninterferon.

EP 0 119 476 A2

Boehringer Ingelheim
International GmbH



Case 12/006
Dr.Fl./Kp.

# Neue Immunglobulin-produzierende Hybridzellinien, deren Verwendung und Verfahren zu deren Herstellung

Es ist bekannt, daß durch die Verfügbarkeit monoklonaler Antikörper die Reinigung von Human-Interferon erleichtert und die Entwicklung von raschen und genauen nicht-biologischen Interferon-Nachweismethoden ermöglicht wird (siehe D. Secher, und D.C. Burke, Nature 285, 446-450 (1980); D.S. Secher, Nature 290, 501-503 (1981); L. Montagnier, A.G. Laurent und J. Gruest, C.R. Acad. Sci Paris 291, Serie D, 893-896 (1980); T. Staehelin, B. Durrer, J. Schmidt, B. Takacs, J. Stocker, V. Miggiano, C. Stähli, M. Rubinstein, W.P. Leuy, R. Hershberg und S. Pestka. Proc. Natl. Acad. Sci, USA 78, 1848-1852 (1981); T. Staehelin, D.S. Hobbs, H. Kung, C.Y. Lai und S. Pestka, J. Biol. Chem. 256, 9750-9754 (1981); J. R. Walker, J. Nagington, G.M. Scott und D.S. Secher, J. gen. Virol. 62, 181-185 (1982)).

Überraschenderweise wurde nun gefunden, daß mit Hilfe neuer Immunglobulin-produzierender Hybridzellinien neue monoklonale Antikörper hergestellt werden können, welche eine hervorragende Antigenspezifität für Interferone des Typs α aufweisen. Außerdem erreichen die Konzentrationen der erfindungsgemäß hergestellten Antikörper im Kulturüberstand 100-200 μg/ml und liegen daher überraschenderweise sehr viel höher als die in der Literatur (siehe beispielsweise G. Galfré, C. Milstein und B. Wright, Nature 277, 131-133 (1979)) als typisch genannten Werte von 1-20 μg/ml.

Gegenstand der vorliegenden Erfindung sind somit neue Immunglobulin-produzierende Hybridzellinien, Verfahren zu deren Herstellung und ein Verfahren zur Herstellung von monoklonalen Immunglobulin G (IgG)-Typ Antikörper der Maus mit selektiver Antigen-Spezifität für Interferone des Typs $\alpha$ bzw. für Subtypen des Humaninterferons $\alpha$.

Die neuen monoklonalen Antikörper sind daher insbesondere zur Hochreinigung und zum Nachweis von Humaninterferon $\alpha$ geeignet.

Erfindungsgemäß erhält man die neuen Immunglobulin-produzierenden Hybridzellinien durch Zellfusion.

Hierzu werden Milzzellen, die unmittelbar nach ihrer Entnahme aus den immunisierten Mäusen, z.B. BALB/c Mäusen, mit Myelomzellen, z.B. Myelomzellen der Linien P3-X-63Ag8-6-5-3, nach der Methode von G. Galfré et al. (siehe Nature 266, 550-552 (1977)) folgendermaßen fusioniert:

Die Milzzellen aus der immunisierten Maus werden mechanisch aus dem Zellverband gelöst und die entstehende Suspension von Zellen wird 1 x mit isotoner Kochsalzlösung gewaschen und zentrifugiert. Die Myelomzellen werden 1 x in serumfreiem Zellkulturmedium gewaschen, bei 1000 x g zentrifugiert und die beiden entstehenden Zellpellets (Myelomzellen und Milzzellen) in serumfreiem Kulturmedium resuspendiert und zusammen bei 1000 x g zentrifugiert. Das Mengenverhältnis beträgt 2 x $10^8$ Myelomzellen zu 2 x $10^8$ Milzzellen. Der Überstand wird nach der Zentrifugation entfernt und die Zellfusionierung in 50 % Polyäthylenglykol , z.B. in 50 % Polyäthylenglykol 4000 der Firma E. Merck, Darmstadt, welches mit 5 % Dimethylsulfoxyd versetzt ist, durchgeführt (1,5 ml Volumen). Nach 2 Minuten Reaktionszeit wird das Reaktionsgemisch mit serumfreiem Medium auf 6 ml verdünnt, anschließend 6 ml Kulturmedium mit 10 % fötalem Rinderserum zugesetzt und die Mischung bei 1000 x g zentrifugiert.

Nach der Fusionierung werden die Zellen in Kulturplatten mit 24 Näpfchen überführt und nach literaturbekannten Methoden, z.B. der Methode von J. W. Littlefield (siehe Science 145, 709 (1964)) in einem Medium, z.B. HAT Medium, welches zweckmäßigerweise mit 2 % foetalem Rinderserum und mit einem geeigneten Antibioticum oder Antibiotica-Gemisch, z.B. mit Penicillin und Streptomycin, angereichert ist, bei 37°C inkubiert.

Die Kulturen, welche Hybridzellwachstum zeigen, werden auf den Gehalt an Interferon-spezifischen Antikörpern auf folgende Weise geprüft:

Aus Kulturüberständen von Näpfen, in denen das Hybridzellwachstum zu mindestens 10-20 % Konfluenz geführt hatte, werden Aliquote von 300 µl entnommen und mit 5 IE rekombinantem Humaninterferon des Subtyps α2(Arg) (hergestellt gemäß europäischer Patentanmeldung Nr. 83 112 812.9 vom 20. 12. 1983) 1-2 Stunden bei 37°C inkubiert. Um den Komplex aus Humaninterferon mit Antikörper auszufällen, wird mit 3 µl Ziegenantiserum gegen Maus Immunglobuline (Calbiochem-Behring) versetzt und weitere 3 Stunden bei 37°C inkubiert. Die Proben werden 5 Minuten in einer Eppendorf Mikrozentrifuge zentrifugiert und die Überstände auf Kulturen von V3 Affennierenzellen, die von G.J. Christofinis etabliert wurden (siehe J. Med. Microbiol. 3, 251-258 (1970)), auf ihren Restgehalt an Interferonaktivität wie folgt getestet:

Die in 24-Napf Gewebekulturplatten mit 500 µl Medium/Napf kultivierten V3-Zellen werden 20 Stunden bei 37°C mit den Testüberständen inkubiert, danach mit etwa 50 pfu/Napf (Plaqueforming units) vesiculärem Stomatitis Virus infiziert und im halbfesten Medium 40 Stunden inkubiert. Nach dieser Zeit wird die Hemmung der Plaquebildung bestimmt.

Das Screenen der spezifischen Antikörperproduktion in den Hybridzellüberständen erfolgt demnach in einem biologischen Test, basierend auf der antiviralen Aktivität des Interferons. Ein einziger Interferon α Subtyp (IFNα2Arg) wird unter der Bedachtnahme verwendet, daß ein monoklonaler Antikörper nur einen Teil der Subtypen im NC-37 oder Leukozyten Interferon bindet und dadurch in letzteren Interferongemischen die gesamte Interferonaktivität nur marginal reduziert.

Für die Herstellung der neuen Hybridzellinien wird folgendes Zell- und Tiermaterial verwendet:

Zur Zellfusionierung wird eine aus der permanent in vitro wachsenden 8-Azaguanin-resistenten Myelomzellinien P3-X-63Ag8 (G. Koehler und C. Milstein, Nature 256, 495 (1975)) abgeleitete Subzellinie P3-X-63Ag8-6-5-3 (J.F. Kearney, A. Radbruch, B. Liesegang und K. Rajewski, J. Immunol. 123, 1548 (1979)) eingesetzt. Diese Subzellinie produziert keine meßbaren Mengen von Immunglobulin und wird in RPMI 1640 Medium, welches mit 10 % foetalem Rinderserum, Penicillin und Streptomycin versetzt ist, kultiviert.

Zur Produktion der Hybridzellinien, welche Immunglobulin mit Anti Human Interferon α Spezifität erzeugen, werden die Mäuse, z.B. BALB/cMäuse, nach folgendem Verfahren immunisiert:

Human Interferon wird nach der Methode von G.R. Adolf, G. Bodo und P. Swetly (siehe Antiviral Res. 1, 257 (1981)) in NC-37 Zellen hergestellt und in einem Reinheitsgrad, welcher

mindestens $10^7$ Internationalen Einheiten Interferon (I.E. IFN)/mg Protein entspricht, in Form einer Emulsion mit komplettem Freund'schem Adjuvans zur Immunisierung von BALB/c Mäusen verwendet.

Immunisierungsschema A:

Die primare Immunisierung jeder Maus einer Gruppe von fünf Tieren wird mit $5 \times 10^6$ IE IFN bei einer Konzentration von $5 \times 10^7$ IE/ml durchgeführt, wobei die Hälfte der Dosis intraperitoneal, der Rest subcutan in 2-3 Stellen am Rücken der Tiere verabreicht wird. Sieben Wochen nach der Erstimmunisierung wird eine Zweitimmunisierung mit $5 \times 10^6$ IE hochgereinigtem Interferon nach gleichem Schema wie für die Erstimmunisierung durchgeführt mit der Ausnahme, daß inkomplettes Freund'sches Adjuvans verwendet wird. Zwei Wochen nach der Zweitimmunisierung werden die Seren der immunisierten Tiere auf den Gehalt an neutralisierenden Antikörpern gegen Interferon aus NC-37 Zellen geprüft. Zu diesem Zeitpunkt haben alle Seren einen Titer von etwa 1000 (Bestimmung siehe Interferon Neutralisationstest). Drei Wochen nach der Zweitimmunisierung erhält eine der Mäuse $5 \times 10^6$ IE des hochgereinigten Interferons ohne Adjuvans intravenös injiziert. Milzzellen werden 4 Tage später für die Zellfusion mit Myelomzellen verwendet.

Immunisierungsschema B:

Eine Gruppe von 5 BALB/c Mäusen wird mit der gleichen Gesamtmenge NC-37 Interferon immunisiert, wobei die Menge von $5 \times 10^6$ IE Interferon auf drei Dosen verteilt in Abständen von 2-3 Wochen injiziert wird. Die Serumtiter liegen nach dieser Zeitspanne zwischen 10 und 200. Nach zusätzlicher Injektion von $5 \times 10^6$ IE hochgereinigtem NC-37 Interferon in 3 Dosen, erreicht der Serumtiter einer Maus 1000, die der anderen Werte von 3000 und höher. Milzzellen von dreien dieser Mäuse werden 19, 22 und 25 Wochen nach der Erstimmunisierung für die Zellfusion mit Myelomzellen eingesetzt. Drei oder vier Tage vor der Milzentnahme erhält die respektive Maus 5 x $10^6$ IE Interferon entweder intraperitoneal oder intravenös.

Interferon Neutralisationstest:

Anti-Interferon Antikörpertiter werden in Serumproben beziehungsweise in Hybridom- oder in Asziteszellkulturüberständen folgenderweise bestimmt:

10 IE Interferon (100 µl) aus NC-37 Zellen werden mit 10 ul von Reihenverdünnungen der Serumproben inkubiert.

Zum Vergleich der Neutralisation verschiedener Interferonproben werden 10 IE Interferon (100 ul) der zu testenden Interferonprobe mit 100 µl monoklonalem Antikörper oder 100 ul einer 1:50 Verdünnung von Kaninchen Antiserum gegen HuIFN-α und/oder gegen HuIFN-ß inkubiert (siehe Methods in Immunology, Basic Methods Justine S. Garvey, Natalie E. Cremer, Dieter H. Sussdorf (1977) W.A. Benjamin Inc.). Nach 60-90 Minuten bei 37°C wird die Rest-Interferonaktivität in den Proben durch Titration auf V3 Zellen bestimmt (Doppelbestimmung).

Erfindungsgemäß erfolgt die Isolierung der neuen monoklonalen Antikörper aus dem Zellkulturüberstand bzw. aus der Aszitesflüssigkeit von tumortragenden Mäusen nach bekannten Methoden, z.B. durch fraktionierte Fällung vorzugsweise mit Ammoniumsulfat und gegebenenfalls anschließende Chromatographie über einen geeigneten Träger, vorzugsweise auf Zellulose-Basis, z.B. Diethylaminoethyl-Zellulose.

Der so hergestellte Antikörper kann nun direkt zum nichtbiologischen Nachweis für Interferon des Typs α bzw. nach seiner konvalenten Bindung an einen biologisch inaktiven Träger zur Hochreinigung von Humaninterferon verwendet werden. Die kovalente Bindung des Antikörpers erfolgt hierbei an einen entsprechend aktivierten Träger, vorzugsweise auf Dextran-Basis, z.B. an CNBr-aktivierte Sepharose oder CH-aktivierte Sepharose der Firma Pharmacia, Uppsala. Zur Hochreinigung wird eine Lösung des Humaninterferons bei schwach basischen pH, z.B. bei einem pH 7-8, über einen so hergestellten Antikörper-Affinitätsträger gepumpt, solange bei pH 7,0 gewaschen bis das Eluat proteinfrei ist, und anschließend das gebundene Interferon im sauren Bereich, z.B. bei pH 2,2, eluiert. Die so erhaltenen proteinhältigen Fraktionen werden, insbesondere für analytische Zwecke, direkt anschließend über einen stark sauren Kationenaustauscher, z.B. den Kationenaustauscher Mono-S der Firma Pharmacia, chromatographiert. Hierbei wird das Humaninterferon des pH 2,2-Eluats sofort von der Kationenaustauscher-Säule absorbiert und anschließend bei einer schwach sauren pH, z.B. bei pH 5,5 ± 0,1, eluiert.

Die nachfolgenden Beispiele sollen die Erfindung näher erläutern:

## Beispiel 1

### Herstellung und Charakterisierung der Hybrid-Zellinie EBI-1

Aus der Zellfusionierung von Milzzellen einer nach dem Schema A immunisierten Maus mit Zellen der Myelomlinie P3-X-63Ag8-6-5-3 wird nach dem beschriebenen Verfahren eine Hybridzellinie isoliert, deren Zellkulturüberstand die antivirale Aktivität von IFN$\alpha$2 (Arg) annähernd vollständig neutralisiert. Diese Hybridzellinie wird EBI-1 bezeichnet und mehrmals subcloniert, um Stabilität der Antikörperproduktion zu gewährleisten. Die Zellinie EBI-1 läßt sich sowohl in Zellkulturmedium in vitro kultivieren, als auch als Tumor nach intraperitonealer Injektion von EBI-1 Zellen in BALB/c Mäusen.

IgG Produktion durch die Hybrid-Zellinie EBI-1:

- Die Hybrid-Zellinie EBI-1 synthetisiert permanent in der Zellkultur (z.B. in RPMI 1640-Medium) Maus IgG1-Anti-HuIFNα-Antikörper, die in das Zellkulturmedium sezerniert werden.
- Die Konzentration dieser sezernierten Antikörper beträgt bis zu 200 ug/ml Zellkulturmedium.
- Die Hybrid-Zellinie EBI-1 wächst auch als intraperitonealer Tumor speziesspezifisch in BALB/c Mäusen und produziert unter diesen Bedingungen ebenfalls Maus IgG1-Anti-HuIFNα-Antikörper.
- Unter diesen in vivo Bedingungen werden 10-20 mg IgG/ml Aszitesflüssigkeit aus den tumortragenden Mäusen gewonnen. Die Konzentration an HuIFNα2 neutralisierenden Antikörpern ist in der Aszitesflüssigkeit etwa um das 100-fache höher als in Kulturüberständen von EBI-1 Zellen.

Wachstum der Hybrid Zellinie EBI-1 in der BALB/c Maus:

| EBI-1 Zellen Applikation | Tage nach der Applikation | Tumorrate | Verdünnung der Aszitesflüssigkeit | Neutralisierung der HuIFNα2 Aktivität |
|---|---|---|---|---|
| intraperitoneal | 14 | 4/5 | $10^{-2}$<br>$10^{-3}$<br>$10^{-4}$<br>$10^{-5}$ | > 99 %<br>> 99 %<br>> 99 %<br>30 % |

Die durch den Tumor produzierten IgG-Antikörper in der Aszitesflüssigkeit der tumortragenden Mäuse wurden in derobigen Tabelle durch Neutralisation der antiviralen Aktivität von HuIFNα2 (rekombinantes Interferon aus E.coli) bestimmt.

Isolierung der Maus IgG-Anti-HuIFNα-Antikörpers der Hybrid-Zellinie EBI-1:

Die Isolierung des aus EBI-1 gewonnenen IgG erfolgt über folgende Reinigungsschritte:

1. Fraktionierte Ammoniumsulfat-Fällung
2. Diethylaminoethyl-Zellulose Chromatographie
   Ausbeute: 100-200 mg/l Zellkulturüberstand.

## Beispiel 2

Charakterisierung der molekularen Eigenschaften des Antikörpers aus EBI-1 Hybridzellen

- Das Molekulargewicht des gereinigten EBI-1-Antikörpers beträgt > 150.000 Dalton (Bestimmung durch Polyacrylamidgelelektrophorese).
- Die spezifische Aktivität des EBI-1-Antikörpers kann durch Ziegen Anti Maus Immunglobulin Antiserum präzipitiert werden.
- Der EBI-1 Antikörper ist vom Typ IgG1, wobei die schwere Kette γ1, die leichte Kette κ ist (Bestimmung durch Ouchterlony Doppeldiffusionsanalyse).
- Der EBI-1 Antikörper wird von Staphylococcus aureus Protein A gebunden.

Molekulare Eigenschaften des EBI-1 Antikörpers:

| | |
|---|---|
| Molekulargewicht: | > 150.000 Dalton |
| Spezies: | Maus |
| Typ: | IgG 1 |
| Schwere Kette: | γ1 |
| Leichte Kette: | κ |

## Beispiel 3

Charakterisierung der biologischen Eigenschaften des EBI-1 Antikörpers. Neutralisierende Wirkung auf antivirale Interferonaktivität

- Der EBI-1 Antikörper besitzt neutralisierende Wirkung auf die antivirale Aktivität von in Escherichia coli produziertem, gereinigtem Humaninterferon des Subtyps α2 (Arg).
- Der EBI-1 Antikörper besitzt partiell neutralisierende Wirkung auf die antivirale Aktivität von natürlichen Gemischen verschiedener Subtypen des humanen α-Interferons, so wie sie in mehreren Zellinien unter unterschiedlichen Bedingungen induziert werden.
- Das Ausmaß der neutralisierenden Aktivität variiert zwischen den einzelnen Subtypen des Interferon α, wie aus der folgenden Abbildung hervorgeht:

EBI-1 ANTIKÖRPER

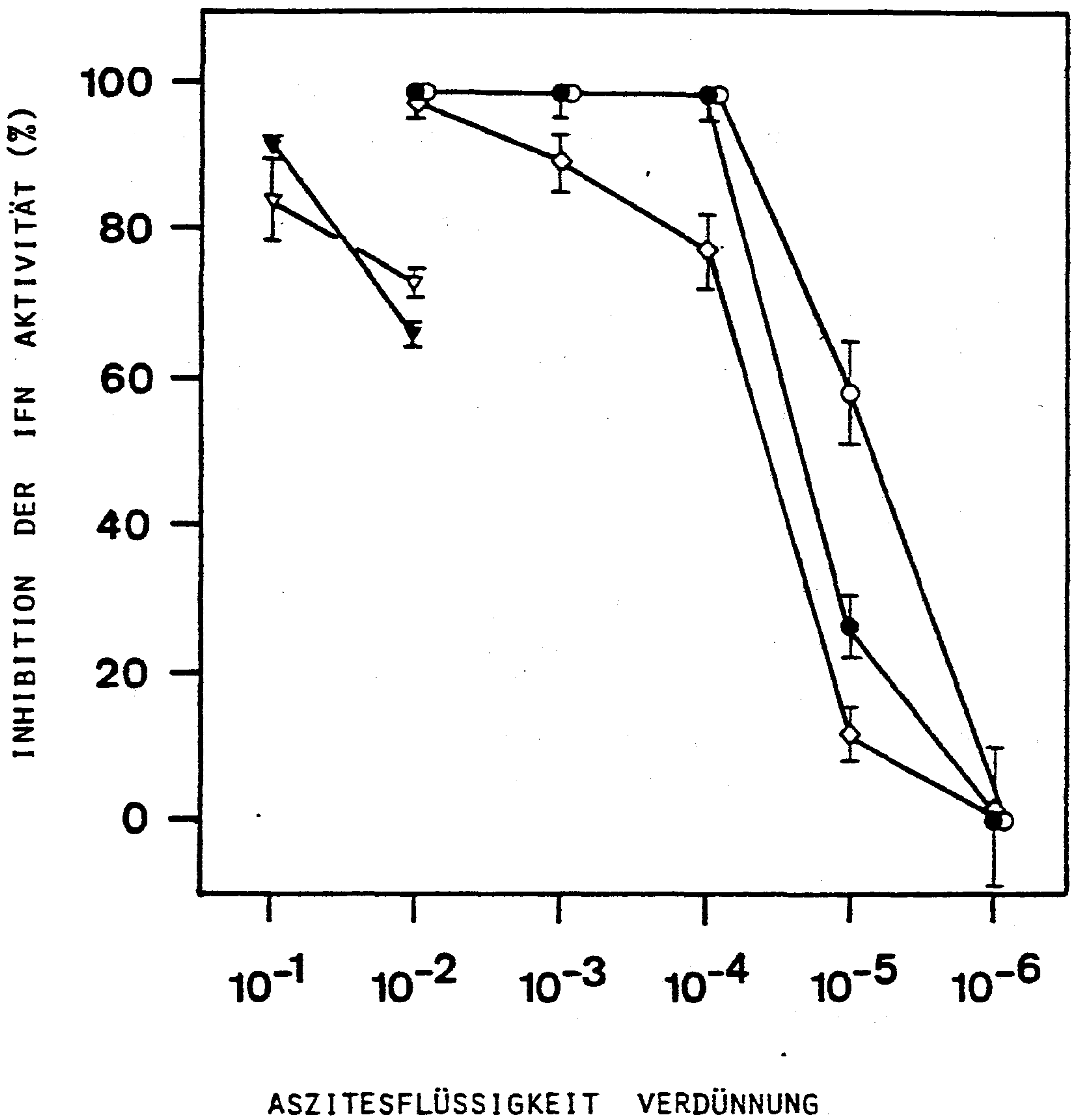


Legende:

Ordinate: Hemmung der Interferonaktivität in %.

Abszisse: Verdünnung der EBI-1 Antikörper Konzentration durch Verdünnung der Aszitesflüssigkeit: Eine Verdünnung $10^{-1}$ entspricht etwa einer Antikörperkonzentration von 1 mg/ml.

L-131

IFN α2 (Arg): Interferon Subtyp α2 (Arg) aus Escherichia coli

IFN αC: Interferon Subtyp αC aus Escherichia coli

Spontan produziertes Interferon aus menschlichen lymphoblastoiden Zellen (Wien-67)

Gemisch von IFN α Subtypen aus Namalwa Zellen

Gemisch von IFN α Subtypen aus menschlichen Blutzellen (Interferon gewonnen nach der Methode von Cantell; = Cantell Interferon)

<u>Zusammenfassung:</u>

- Der EBI-1 Antikörper besitzt keine neutralisierende Aktivität gegen Humaninterferon ß.
- Der EBI-1 Antikörper besitzt keine neutralisierende Aktivität gegen Humaninterferon γ.
- Der EBI-1 Antikörper besitzt keine neutralisierende Aktivität gegen Mausinterferon.
- Der EBI-1 Antikörper eignet sich demnach zum analytischen Nachweis von Humaninterferon der Subtypen α, zur Differenzierung der Subtypen und zur Unterscheidung von anderen Humaninterferonen und Interferonen aus anderen Spezies.

Besonders ausgeprägt ist die neutralisierende Eigenschaft des EBI-1 Antikörpers gegen Interferon des Subtyps α2 und gegen α-Typ Interferon, welches spontan in humanen lymphoblastoiden Zellen (z.B. Wien-67) gebildet wird. Dieses Spontan-Interferon wird in der Zellkultur ohne Zusatz von Interferon induzierenden Agentien wie Sendai Virus oder Poly(I):(C) produziert und von den Zellen kontinuierlich in das Kulturmedium freigesetzt.

Etwas schwächer ist die neutralisierende Wirkung der EBI-1 Antikörper gegen die antivirale Aktivität des Interferons αC. Bei zehntausendfacher Verdünnung der EBI-1 Aszitesflüssigkeit werden noch 77 %, bei hunderttausendfacher Verdünnung 15 % der antiviralen Aktivität neutralisiert.

Die schwächste Wirkung wird bei der Neutralisation der antiviralen Aktivität aus Namalwa Zellen (Gemisch aus mindestens fünf IFNα Subtypen) und aus Blutzellen (Cantell Interferon: Gemisch aus 7-8 IFNα Subtypen) beobachtet. Bei einer hundertfachen Aszitesverdünnung werden nur 60-80 % der antiviralen Aktivität des Interferongemisches neutralisiert.

Die nachfolgende Tabelle zeigt die Neutralisationsergebnisse durch EBI-1-Antikörper für weitere Humaninterferonpräparationen, welche aus unterschiedlichen Zelltypen erhalten werden und durch Behandlung der Zellen oder durch Induktionsverfahren in ihrer Zusammensetzung modifiziert worden sind:

| Herkunft des Interferons | | | % IFN Aktivität neutralisiert durch: | | | |
|---|---|---|---|---|---|---|
| Zellen | Behandlung der Zellen | Interferon Induktor | EBI-1 AK | Kaninchen anti IFN-α | EBI-1 AK + Kaninchen anti IFN-α | Kaninchen anti IFN-α + anti IFN-β |
| E.coli (HuIFNα2) (Arg) | keine | keinen | > 99 | > 99 | - | - |
| periphäre Blutleukozyten (gesunde Spender) | keine | Sendai-Virus | 65 | > 99 | - | - |
| periphäre Blutleukozyten (leukämische Spender) | keine | Sendai-Virus | 67 | > 99 | - | - |
| Namalwa | keine | Sendai-Virus | 64 | 81 | 71 | > 98 |
| | Butyrat | Sendai-Virus | 72 | > 98 | - | - |
| | Butyrat | poly(I):(C) | 76 | > 97 | - | - |
| NC-37 | keine | Sendai-Virus | 18 | 55 | 75 | > 98 |
| | keine | poly(I):(C) | 49 | 82 | - | > 95 |
| | Butyrat | Sendai-Virus | 65 | > 97 | - | - |
| | BrdUrd | keinen | > 98 | > 98 | - | - |
| NSL-246/79 | keine | Sendai-Virus | 75 | > 97 | - | - |
| | keine | keinen | > 98 | > 98 | - | - |
| Wien-62 | keine | Sendai-Virus | 56 | > 97 | - | - |
| | keine | keinen | > 98 | > 98 | - | - |
| | BrdUrd | keinen | > 97 | > 97 | - | - |
| Wien-67 | keine | Sendai-Virus | 63 | > 97 | - | - |
| | keine | keinen | > 98 | > 97 | - | - |
| | BrdUrd | keinen | > 97 | > 97 | - | - |
| Wien-142, Wien-143 Wien-146 | keine | keinen | > 95 | > 95 | - | - |

Legende zu der vorstehenden Tabelle:

Abkürzungen:

AK - Antikörper

E. coli HuIFNα2 - rekombinantes Interferon des Typs α2 (Arg) aus Escherichia coli Kulturen.

BrdUrd - Bromdeoxyuridin.

poly(I):(C) - poly Inosin: poly Cytidin.

Die Zellinien "Namalwa" und "NC-37" sind von Burkitt's Lymphomen abgeleitet, die anderen Zellinien aus Blutlymphozyten, die entweder mit B95/8 Epstein Barr Virus (NSL-246/79) oder durch spontane Transformation etabliert worden sind (Wien 62, 67, 142, 143, 146).

Die Neutralisationstests werden mit Überständen aus EBI-1 Zellkulturen oder aus 1:100 verdünnter Aszitesflüssigkeit als Quelle für die Antikörper durchgeführt. Mit beiden Antikörper Präparationen werden identische Resultate erzielt.

Mindestens zwei unabhängige Neutralisationsbestimmungen werden für jede angegebene Bestimmung durchgeführt und die Mittelwerte daraus sind in der vorstehenden Tabelle angegeben.

Zusammenfassung:

Aus der unterschiedlichen Vorbehandlung der Zellen (z.B. der Typen Namalwa, NC-37, NSL-246/79, Wien-62, Wien-67) resultiert eine unterschiedliche Komposition des Interferon α-Subtypen-Gemisches, was sich im jeweiligen Prozentsatz der durch EBI-1 Antikörper neutralisierten antiviralen Aktivität spiegelt.

In jenen Zellen in denen zusätzlich zu einem Gemisch von Interferon α Subtypen auch Interferon ß induziert wird, kann durch den EBI-1 Antikörper das Interferon ß nicht neutralisiert werden.

Beispiel 4

Charakterisierung der biologischen Eigenschaften des EBI-1-Antikörpers: Bindung von Interferon

a) Kovalente Bindung des Antikörpers an biologisch inertes Trägermaterial.
Als inertes Trägermaterial wird sowohl CNBr-aktivierte Sepharose 4B als auch aktivierte CH-Sepharose 4B, der Firma Pharmacia, eingesetzt. Für jeweils 1 g des Trägermaterials werden 25 mg gereinigter EBI-1-Antikörper eingesetzt. Die Kupplung des EBI-1-Antikörpers an den Träger erfolgt in 0,2 M $NaHCO_3$ + 0,5 M NaCl (pH 8,4) bei Zimmertemperatur 2 Stunden lang. Danach wird das Trägermaterial abgesaugt, mit 1 M Ethanolamin, pH 8,0 1 Stunde lang zur Blockierung restlicher aktiver Gruppen inkubiert und durch 3 Waschzyklen bei pH 4,0 (0,1 M Azetat + 1 M NaCl) und pH 8 (0,1 M Borat + 1 M NaCl) aufeinanderfolgend gewaschen. Der fertige Affinitätsträger wird bei pH 7,0 (0,02 Na-Phosphat + 0,15 M NaCl) unter Zugabe von 0,1 % Natriumazid bei Kühlraumtemperatur gelagert.

b) Reversible Bindung von Humaninterferon-α an den Affinitätsträger.
Die IFN-α-Lösung wird bei pH 7-8 und einer Ionenstärke von 0,1 - 0,5 M NaCl durch die Affinitätssäule gepumpt. Das Interferon wird von der Säule gebunden. Die Säule wird sodann mit 0,02 M Na-Phosphat + 0,15 m NaCl, pH 7,0 solange gewaschen, bis das Eluat proteinfrei ist. Die Elution des gebundenen Interferons wird mit Hilfe von 0,1 M Zitronensäure in 25 % (Vol/vol) Ethylenglykol bei pH 2,2 vorgenommen. Das Eluat wird fraktioniert und die proteinhältigen Fraktionen gesammelt.

Die nachfolgenden Tabellen enthalten die für IFN-α2 (Arg) aus E. coli und IFN-α Gemisch aus Namalwa Zellen erhaltenen Ergebnisse:

a) IFN-α2 (Arg) aus E.coli
Träger: EBI-1-Sepharose 4B
Bettvolumen 6 ml

| | Einheiten IFN | (%) | Einheiten IFN/mg Protein | |
|---|---|---|---|---|
| Aufgetragen | $1{,}20 \times 10^9$ | (100) | $2{,}1 \times 10^6$ | |
| Nicht absorbiert | $0{,}17 \times 10^9$ | ( 14) | $0{,}4 \times 10^6$ | |
| IFN im sauren Eluat | $1{,}10 \times 10^9$ | ( 92) | $380 \times 10^6$ | (x 180) |

b) IFN-α Gemisch aus Namalwa Zellen
Träger: EBI-1 Antikörper-CH-Sepharose 4B
Bettvolumen: 3 ml

| | Einheiten IFN | (%) | Einheiten IFN/mg Protein | |
|---|---|---|---|---|
| Aufgetragen | $145 \times 10^6$ | (100) | $0{,}078 \times 10^6$ | |
| Nicht absorbiert | $10 \times 10^6$ | ( 7) | $0{,}005 \times 10^6$ | |
| IFN im sauren Eluat | $127 \times 10^6$ | ( 88) | $127 \times 10^6$ | (x 1630) |

## Beispiel 5

**Anwendung des EBI-1-Antikörper-Affinitätsträgers für die Reinigung in Verbindung mit anschließender Chromatographie an MONO S-Säulen des Pharmacia FPLC-Systems**

Für die weitere Reinigung des IFN-α, das im sauren Eluat der EBI-1-Antikörper-Affinitätssäulen enthalten ist, eignet sich die Chromatographie am stark sauren Kationenaustauscher

MONO-S der Firma Pharmacia aus folgenden Gründen:
1. Das saure Eluat kann ohne weitere Manipulationen, wie z.B. Dialyse, zur Chromatographie eingesetzt werden.

2. Die Kombination EBI-1-Antikörper-Affinitätschromatographie mit MONO S-Chromatographie ergibt IFN-α Präparate von hoher Reinheit. Das Eluat der EBI-1 Antikörper-Affinitätssäule wird sofort durch die MONO S-Säule vom Typ HR 5/5 gepumpt und das IFN an der MONO S-Säule absorbiert. Die Elution erfolgt durch Anwendung eines pH-Gradienten. Dieser wird durch Mischen zweier Pufferlösungen hergestellt.

Puffer A: 0,1 M Na-Zitrat in 25 % (v/v) Propandiol-1,2 (pH 4,5)
Puffer B: 0,1 M Na-Phosphat in 25 % (v/v) Propandiol-1,2 (pH 8,0)
Das IFN wird als scharfer Peak eluiert und zwar bei pH 5,50 + 0,10, was durch Messung der Extinktion des Eluates bei 280 nm feststellbar ist. Das Eluat wird fraktioniert und die entsprechenden Fraktionen werden gepoolt.

Die nachfolgenden Tabellen enthalten die für IFN-α2 (Arg) aus E. coli und IFN-α Gemisch aus NC-37-Zellen erhaltenen Ergebnisse:

a) IFN $\alpha 2$ (Arg) aus E.coli

EBI-1-Antikörper-Säule: Träger CH-Sepharose 4B, Bettvolumen 50 ml

MONO S-Säule: Typ HR 5/5 (Vol 1 ml), angeschlossen an das FPLC-System von Pharmacia.

| | Einheiten IFN | (%) | Einheiten IFN/mg Protein | (Reinigung) |
|---|---|---|---|---|
| IFN vor EBI-1-Antikörper Chromatographie | $4,13 \times 10^9$ | (100,0) | $0,82 \times 10^6$ | (x 385) |
| IFN im Eluat der EBI-1-Antikörper-Säule | $3,91 \times 10^9$ | ( 94,6) | $316 \times 10^6$ | (x 385) |
| IFN pH 5,5-Peak MONO-S-Säule | $2,46 \times 10^9$ | ( 59,6) | $515 \times 10^6$ | (x 628) |

b) IFN $\alpha$ Gemisch aus Butyrat-behandelten und Sendaivirusinduzierten NC-37 Zellen

EBI-1-Antikörper-Säule: Träger CH-Sepharose 4B, Bettvolumen 5 ml

MONO S-Säule: Typ HR 5/5 (Vol. 1 ml), angeschlossen an das FPLC-System von Pharmacia

| | Einheiten IFN | (%) | Einheiten IFN/mg Protein (Reinigung) | |
|---|---|---|---|---|
| IFN vor EBI-1-Antikörper-Chromatographie | 90,6 x $10^6$ | (100) | 0,041 x $10^6$ | |
| IFN im Eluat der EBI-1-Antikörper-Säule | 83,9 x $10^6$ | ( 93) | 60 x $10^6$ | (x 1460) |
| IFN pH 5,5-Peak MONO-S-Säule | 47,4 x $10^6$ | ( 52) | 180 x $10^6$ | (x 4390) |

Der pH 5,5-Peak in dem Beispiel NC-37 IFN α ist unsymmetrisch und deutet auf Heterogenität des IFN α aus diesen Zellen hin.

## Beispiel 6

### Herstellung und Charakterisierung der Hybrid Zellinie EBI-2

Aus der Zellfusionierung von Milzzellen einer nach dem Schema B immunisierten Maus mit Zellen der Myelomlinie P3-X-63Ag8-6-5-3 wird nach dem beschriebenen Verfahren eine Hybridzellinie isoliert, deren Zellkulturüberstände die antivirale Aktivität von IFNα2 (Arg) annähernd vollständig neutralisieren. Diese Hybridzellinie wird EBI-2 bezeichnet und mehrmals subcloniert, um die Stabilität der Antikörperproduktion zu gewährleisten. Die Zellinie EBI-2 läßt sich sowohl in Zellkulturmedium in vitro kultivieren als auch als Tumor nach intraperitonealer Injektion von EBI-2 Zellen in BALB/c Mäusen.

IgG Produktion durch die Hybridzellinie EBI-2:

Die Hybridzellinie EBI-2 synthetisiert permanent in der Zellkultur (z.B. in RPMI 1640 Medium mit 10 % foetalem Rinderserum) Maus IgG1-Anti-HuIFN-α-Antikörper, die in das Zellkulturmedium sezerniert werden. Die Konzentration dieser sezernierten Antikörper beträgt bis zu 100 µg/ml Zellkulturmedium.

Die Hybridzellinie EBI-2 wächst auch als intraperitonealer Tumor speziespezifisch in BALB/c Mäusen und produziert unter diesen Bedingungen gleichfalls Maus IgG1-Anti-HuIFN-αAntikörper. Unter diesen Bedingungen werden 10-15 mg IgG/ml Aszitesflüssigkeit aus den tumortragenden Mäusen gewonnen.

Wachstum der Hybridzellinie EBI-2 in der BALB/c Maus

| EBI-2 Zellen Applikation | Tage nach der Applikation | Tumorrate | Verdünnung der Aszitesflüssigkeit | Neutralisation der HuIFNα2 (Arg) Aktivität |
|---|---|---|---|---|
| $10^7$ Zellen intraperitoneal | 15 | 4/5 | $10^1$ | > 98 % |
| | | | $10^2$ | > 98 % |
| | | | $10^3$ | > 98 % |
| | | | $10^4$ | 80 % |
| | | | $10^5$ | 24 % |

Die durch den Tumor produzierten IgG Antikörper in der Aszitesflüssigkeit der tumortragenden Mäuse wurden in der obigen Tabelle durch Neutralisation der antiviralen Aktivität von HuIFNα2 (Arg) (rekombinantes Interferon aus E.coli) bestimmt.

Isolierung des Maus IgG Anti HuIFNα Antikörpers der Hybridzellinie EBI-2

Die Isolierung des EBI-2 Antikörpers erfolgt über folgende Reinigungsschritte:
1. Fraktionierte Ammonsulfatfällung
2. Diethylaminoethyl-Zellulose Chromatographie
Die Ausbeute beträgt 100 - 150 mg IgG/l Zellkulturüberstand.

## Beispiel 7

Charakterisierung der molekularen Eigenschaften des Antikörpers aus EBI-2 Hybridzellen

Das Molekulargewicht des gereinigten EBI-2 Antikörpers beträgt > 150.000 Dalton (Bestimmung durch Polyacrylamidgelelektrophorese).

Der EBI-2 Antikörper ist vom Typ IgG1, wobei die schwere Kette γ1, die leichte Kette κ ist (Bestimmung durch Ouchterlony Doppeldiffusionsanalyse). Der EBI-2 Antikörper wird durch Staphylococcus aureus Protein A gebunden. Die Aktivität des EBI-2 Antikörpers kann durch Ziegen-Anti Maus Immunglobulin Antiserum präzipitiert werden.

Molekulare Eigenschaften des EBI-2 Antikörpers:

| | |
|---|---|
| Molekulargewicht: | > 150.000 Dalton |
| Spezies | Maus |
| Typ | IgG1 |
| Schwere Kette | γ1 |
| leichte Kette | κ |

## Beispiel 8

Charakterisierung der biologischen Eigenschaften des Antikörpers aus EBI-2 Hybridzellen

Der EBI-2 Antikörper besitzt neutralisierende Wirkung auf die antivirale Aktivität von in E.coli produziertem, gereinigtem Humaninterferon des Subtyps α2 (Arg). Eine tausendfache Verdünnung der Aszitesflüssigkeit aus EBI-2 tumortragenden BALB/c Mäusen neutralisiert IFNα2 beinahe vollständig, eine zehntausendfache Verdünnung zu 80 % und eine hunderttausendfache Verdünnung zu 24 %.

Der EBI-2 Antikörper besitzt neutralisierende Wirkung auf die antivirale Aktivität von spontan produziertem Interferon. Bei einer Verdünnung der Aszitesflüssigkeit bis zum zehntausendfachen ist die Neutralisation gleichartig wie bei Interferon des Subtyps α2 (Arg). Bei einer Aszitesverdünnung von EBI-2 auf das hunderttausendfache werden noch etwa 50 % des spontan produzierten Interferons aus Wien-67 Zellen neutralisiert.

Ein völlig anderes Wirkungsspektrum des EBI-2 Antikörpers im Vergleich zum EBI-1 Antikörper zeigt sich bei der Neutralisation von Interferon des Subtyps αC aus Escherichia coli und von Namalwa- und Blutzellen (Cantell) Interferon Gemischen: IFNαC-Aktivität wird auch durch konzentrierte Asziteslösung (Konzentration des EBI-2 Antikörpers > 1 mg/ml) nicht neutralisiert; Interferongemisch aus Blutzellen nur zu 30 % und Interferongemisch aus Namalwa Zellen nur zu etwa 50 %.

Das Wirkungsspektrum der EBI-2 Antikörper gegen einige Interferonpräparationen ist in der folgenden Abbildung dargestellt:

EBI-2 ANTIKÖRPER

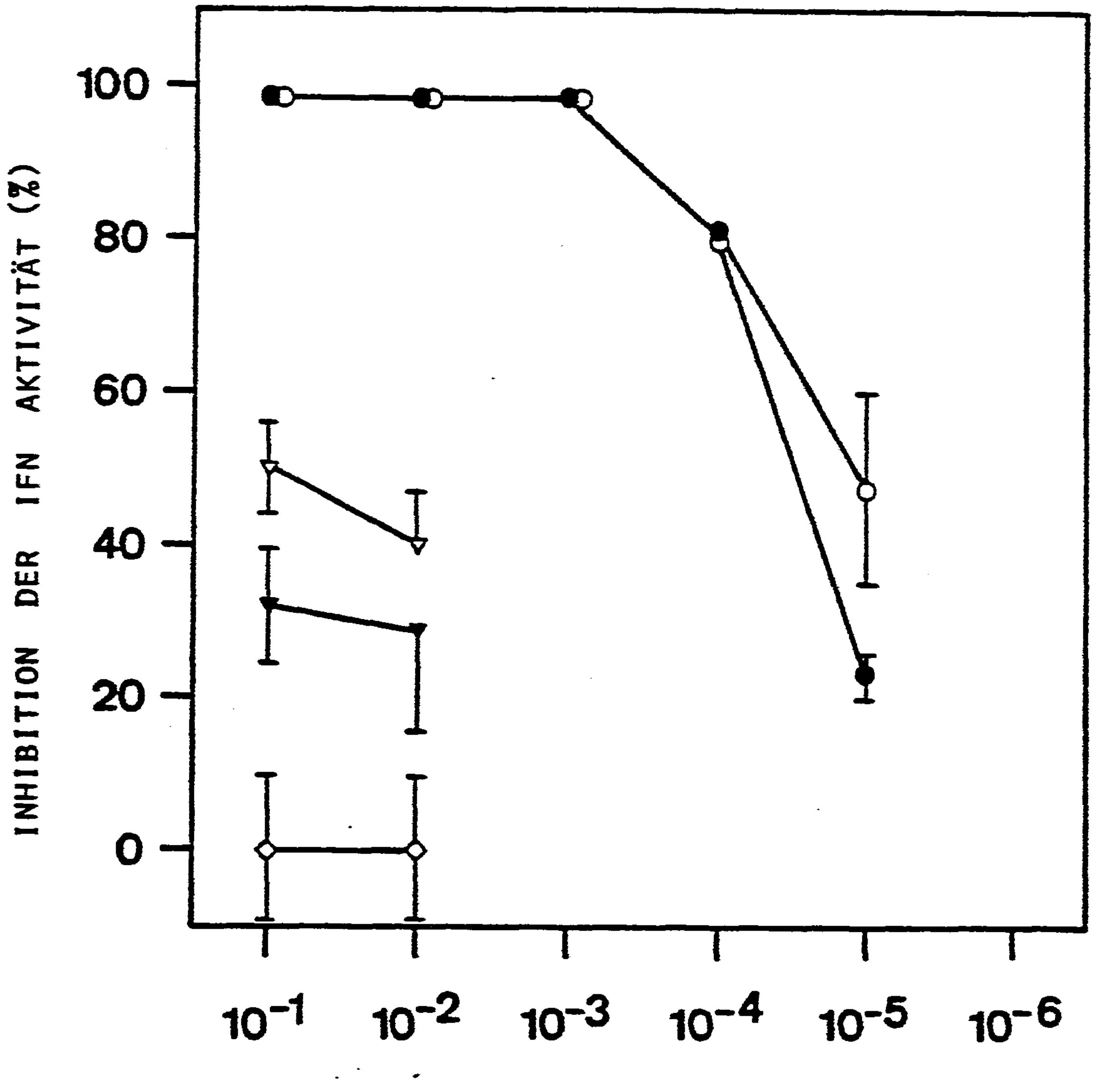


Legende:

Ordinate: Hemmung der Interferonaktivität in %

Abszisse: Verdünnungsreihe der EBI-2 Antikörperkonzentration durch Verdünnung des Aszitesflüssigkeit: Eine Verdünnung $10^{-1}$ entspricht etwa einer Antikörperkonzentration von 1 mg/ml.

0119476

●——● IFN $\alpha$2 (Arg): Interferon Subtyp $\alpha$2 (Arg) aus Escherichia coli

◇——◇ IFN $\alpha$C: Interferon Subtyp $\alpha$C aus Escherichia coli

○——○ Spontan produziertes Interferon aus menschlichen lymphoblastoiden Zellen (Wien-67)

▽——▽ Gemisch von IFN $\alpha$ Subtypen aus induzierten Namalwa Zellen

▼——▼ Gemisch von IFN $\alpha$ Subtypen aus induzierten menschlichen Blutzellen (Cantell Interferon)

## Beispiel 9

### Herstellung und Charakterisierung der Hybrid Zellinie EBI-3

Aus der Zellfusionierung von Milzzellen einer nach dem Schema B immunisierten Maus mit Zellen der Myelomlinie P3-X-63Ag8-6-5-3 wird nach dem beschriebenen Verfahren eine Hybridzellinie isoliert, deren Zellkulturüberstände die antivirale Aktivität von IFN$\alpha$2 (Arg) annähernd vollständig neutralisieren. Diese Hybridzellinie wird EBI-3 bezeichnet und mehrmals subkloniert, um die Stabilität der Antikörperproduktion zu gewährleisten. Die Zellinie EBI-3 läßt sich sowohl in Zellkulturmedium in vitro kultivieren als auch als Tumor nach intraperitonealer Injektion von EBI-3 Zellen in BALB/c Mäusen.

### Immunglobulinproduktion durch die Hybridzellen EBI-3:

Die Hybridzellinie EBI-3 synthetisiert permanent in der

Zellkultur (z.B. in RPMI 1640 Medium mit 10 % foetalem Rinderserum) Maus IgG3 Anti-HuIFN α Antikörper, die Menge der in das Zellkulturmedium sezernierten Antikörper beträgt bis zu 100 ug/ml Zellkulturüberstand.

Die Hybridzellinie EBI-3 wächst auch als intraperitonealer Tumor speziespezifisch in BALB/c Mäusen und produziert unter diesen Bedingungen gleichfalls Maus IgG3- Anti-Humaninterferon α Antikörper. Unter diesen Bedingungen werden 10-15 mg IgG/ml Aszitesflüssigkeit aus den tumortragenden Mäusen gewonnen.

Wachstum der Hybridzellen EBI-3 in der BALB/c Maus

| EBI-3 Zellen Applikation | Tage nach der Applikation | Tumor rate | Verdünnung der Aszitesflüssigkeit | Neutralisation der HuIFNα2 (Arg) Aktivität |
|---|---|---|---|---|
| $10^7$ Zellen intraperitoneal | 14 | 4/4 | $10^{-1}$ | ≥ 98 % |
| | | | $10^{-2}$ | ≥ 98 % |
| | | | $10^{-3}$ | ≥ 98 % |
| | | | $10^{-4}$ | ≥ 98 % |
| | | | $10^{-5}$ | 43 % |

Die durch den Tumor produzierten IgG-Anti-Antikörper in der Aszitesflüssigkeit der tumortragenden Mäuse wurden in der obigen Tabelle durch Neutralisation der antiviralen Aktivität von HuIFNα2 (Arg) (rekombinantes Interferon des Subtyps IFNα2 (Arg) aus Escherichia coli) bestimmt.

Isolierung des Maus IgG Anti HuIFNα Antikörpers der Hybridzellinie EBI-3

Die Isolierung des EBI-3 Antikörpers erfolgt über folgende Reinigungsschritte:

1. Fraktionierte Ammonsulfatfällung
2. Diethylaminoethyl-Zellulose Chromatographie

Die Ausbeute beträgt 100 - 150 mg IgG/l Zellkulturüberstand.

Beispiel 10

Charakterisierung der molekularen Eigenschaften des Antikörpers, welcher von EBI-3 Zellen produziert wird (EBI-3 Antikörper)

Das Molekulargewicht des gereinigten EBI-3 Antikörpers beträgt ≥ 150.000 Dalton (Bestimmung durch Polyacrylamidelektrophorese).
Der EBI-3 Antikörper ist vom Typ IgG3, wobei die schwere Kette γ3, die leichte Kette κ ist (Bestimmung durch Ouchterlony Doppeldiffusionsanalyse).
Der EBI-3 Antikörper wird durch Staphylococcus aureus Protein A gebunden.
Die Aktivität des EBI-3 Antikörpers wird durch Ziegen-Anti-Maus-Immunglobulin Antiserum präzipiert.

Molekulare Eigenschaften des EBI-3 Antikörpers

| | |
|---|---|
| Molekulargewicht | ≥ 150.000 Dalton |
| Spezies | Maus |
| Typ | IgG3 |
| Schwere Kette | γ3 |
| Leichte Kette | κ |

## Beispiel 11

Charakterisierung der biologischen Eigenschaften des EBI-3 Antikörpers

Der EBI-3 Antikörper besitzt neutralisierende Wirkung auf die antivirale Aktivität von in E.coli produziertem gereinigtem Humaninterferon des Subtyps α2 (Arg). Eine zehntausendfache Verdünnung der Aszitesflüssigkeit aus EBI-3 tumortragenden BALB/c Mäusen neutralisiert IFNα2 (Arg) beinahe vollständig, eine hunderttausendfache Verdünnung zu 43 %.

Im gleichen Ausmaß besitzt der EBI-3 Antikörper neutralisierende Wirkung auf spontan in Wien-67 Zellen produziertes Interferon (Spontan bedeutet Interferon, welches ohne Zusätze von Interferoninduktoren von Zellen gebildet wird). Der EBI-3 Antikörper neutralisiert Interferon Subtyp αC aus Escherichia coli, wobei die Wirkung jedoch unterschiedlich von der des EBI-1 Antikörpers ist.

Die Interferongemische aus Sendai Virus induzierten Namalwa Zellen und Sendai Virus induzierten Blutzellen (Cantell Interferon) werden zu etwa 60 % von einer hundertfachen Verdünnung der EBI-3 Aszitesflüssigkeit neutralisiert.

Das Wirkungsspektrum der EBI-3 Antikörper gegen einige Interferon Präparationen ist in der folgenden Abbildung dargestellt:

EBI-3 ANTIKÖRPER

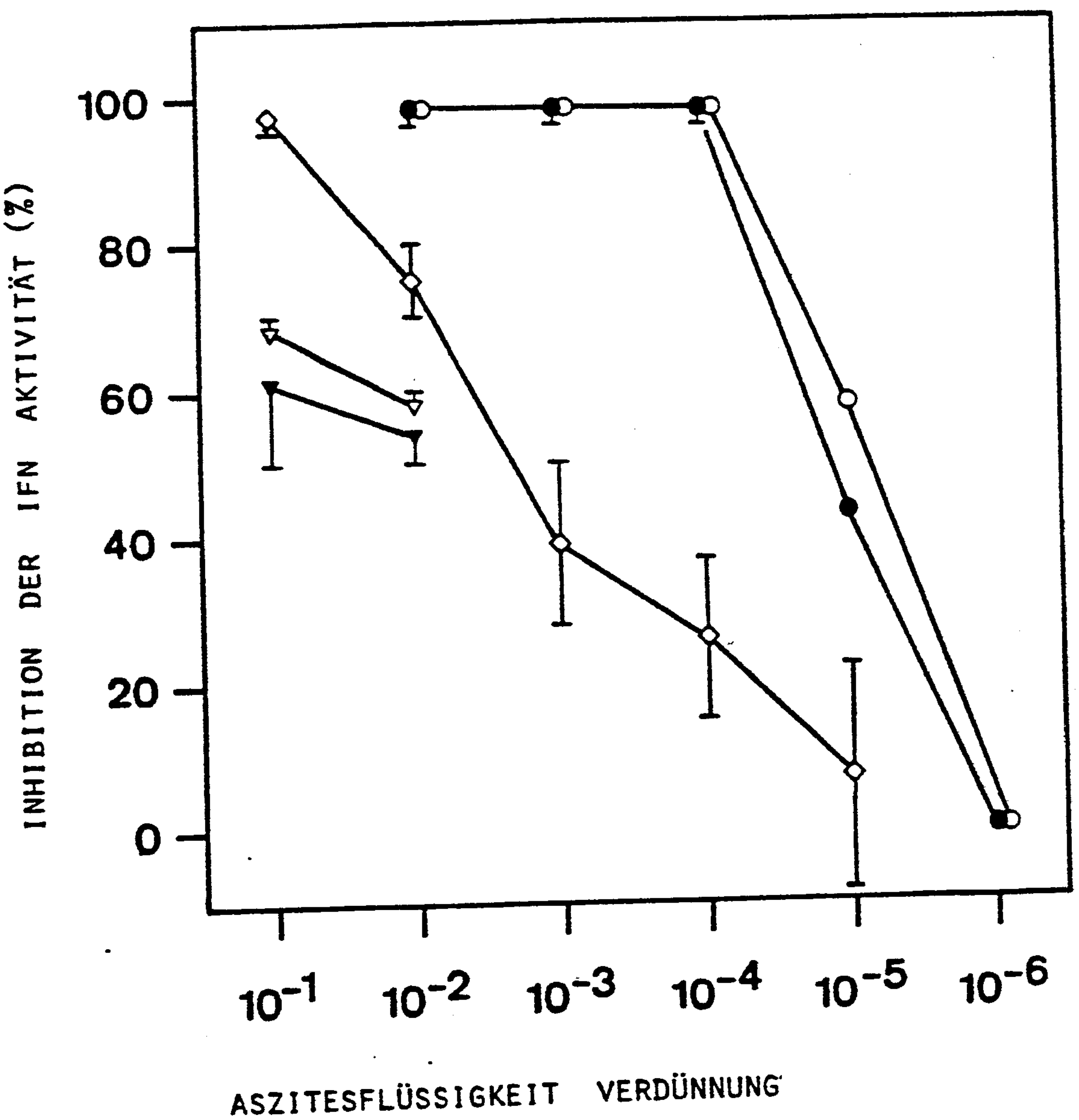


<u>Legende:</u>

Ordinate: Hemmung der Interferonaktivität in %

Abszisse: Verdünnungsreihe der EBI-3 Antikörperkonzentration durch Verdünnung der Aszitesflüssigkeit: Eine Verdünnung $10^{-1}$ entspricht etwa einer Antikörperkonzentration von 1 mg/ml.

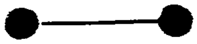 IFN $\alpha 2$ (Arg): Interferon Subtyp $\alpha 2$ (Arg) aus Escherichia coli

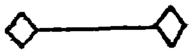 IFN $\alpha$C: Interferon Subtyps $\alpha$C aus Escherichia coli.

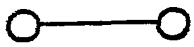 Spontan produziertes Interferon aus menschlichen lymphoblastoiden Zellen (Wien 67)

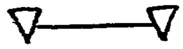 Gemisch von IFN $\alpha$ Subtypen aus induzierten Namalwa-Zellen

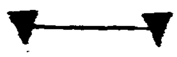 Gemisch von IFN $\alpha$ Subtypen aus induzierten menschlichen Blutzellen (Cantell Interferon)

Beispiel 12

Trennung von Subtypen des Humaninterferon $\alpha$ aus Namalwa Zellen und deren Charakterisierung mit Hilfe der monoklonalen Antikörper EBI-1, EBI-2 und EBI-3

Trennung der Subtypen:

Prinzip: Chromatofokussierung an MONO P-Säulen (MONO P-HR5/20, Fa. Pharmacia, Uppsala) im pH-Bereich von 7,1 - 4,0.

Das Subtypen-Gemisch wird gegen 0,025 M Bis-Tris (Sigma B 9754) - Imino-Diessigsäure (Sigma I 5629), pH 7,1 in 25 % v/v 1,2-Propandiol-Wasser dialysiert und an der MONO P-Säule adsorbiert. Die Säule ist an das FPLC-System von Pharmacia angeschlossen. Das an der Säule absorbierte Subtypengemisch wird durch einen pH-Gradienten eluiert. Hierzu wird Polypuffer 74 (Pharmacia) in 25 % v/v 1,2-Propandiol mit Imino-Diessigsäure auf pH 4,0 eingestellt und dieses Gemisch durch die MONO P-Säule gepumpt. Das Eluat wird fraktioniert und pH-Wert sowie Interferon-Gehalt der Fraktion bestimmt.

Die folgende Abbildung A zeigt die Auftrennung der IFN-α Subtypen aus einem von Sendai Virus-infizierten Namalwa-Zellen produziertem Gemisch:

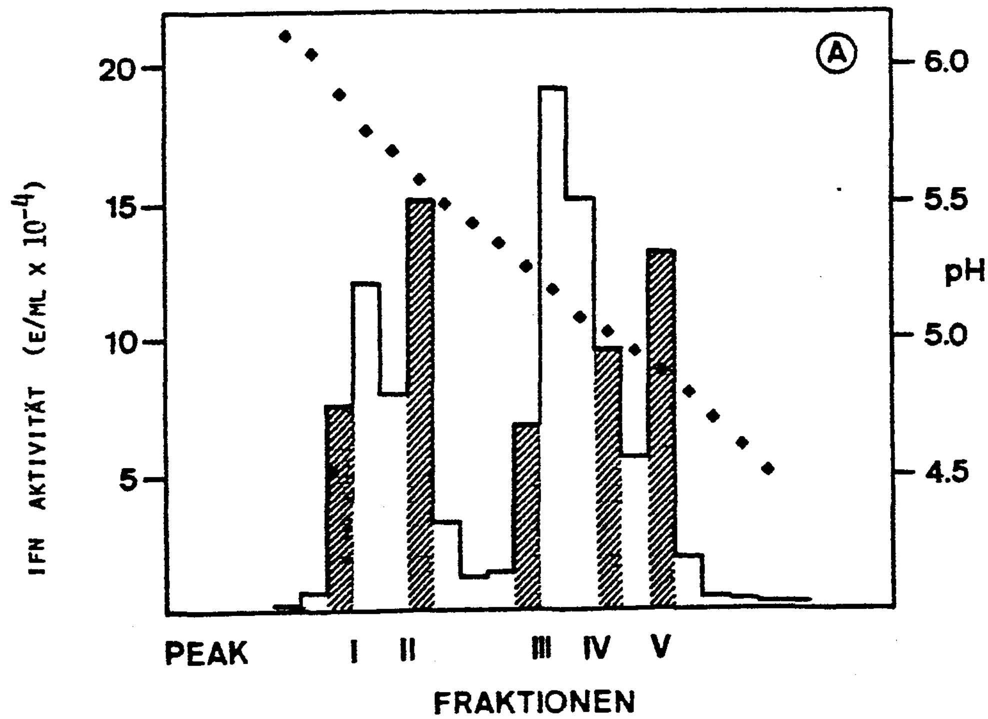


Hiervon werden 5 Fraktionen ausgewählt (siehe schraffierte Fraktionen in Abbildung A) und im Neutralisationstest, wie eingangs beschrieben, mit den monoklonalen Antikörpern EBI-1, EBI-2 und EBI-3 eingesetzt. Die folgende Abbildung B zeigt, daß die einzelnen Komponenten in charakteristischer Weise mit den Antikörpern reagieren:

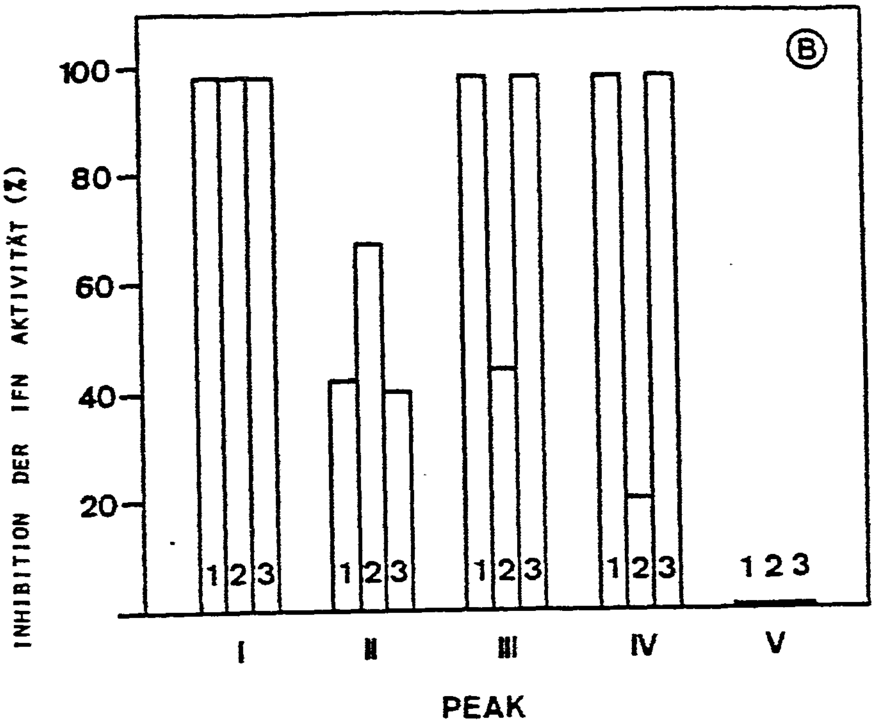


Zusammenfassung:

1. EBI-1, -2 und -3 Antikörper hemmen zu annähernd 100 % die antivirale Aktivität desjenigen Subtyps von IFNα, welcher bei pH 5,8 von der MONO P-Säule eluiert wird.

2. EBI-1, -2 und 3-Antikörper hemmen zu 42, 67 und 40 %, respektive, die antivirale Aktivität des IFNα Subtyps, welcher bei pH 5,5 von der MONO P-Säule eluiert wird.

3. EBI-1, -2 und 3-Antikörper hemmen zu 98, 44 und 98 % respektive die antivirale Aktivität desjenigen IFNα Subtyps, welcher bei pH 5,25 von der MONO P-Säule eluiert wird.

4. EBI-1, -2 und -3 Antikörper hemmen zu 98, 20 und 98 % respektive die antivirale Aktivität desjenigen IFNα Subtyps, welcher bei 5,0 von der MONO P-Säule eluiert wird.

5. Weder EBI-1, -2 noch EBI-3 Antikörper hemmen die antivirale Aktivität desjenigen IFNα Subtyps, welcher bei pH 4,8 von der MONO P-Säule eluiert wird.

0119476

## Patentansprüche

1. In vitro und in vivo zu kultivierende Hybridzellinie, dadurch gekennzeichnet, daß sie durch Zellfusion von Milzzellen einer mit Humaninterferon des Typs $\alpha$ immunisierten Maus mit Myelomzellen erhalten wird und Antikörper gegen Humaninterferon des Typs $\alpha$ produziert.

2. Hybridzellinie gemäß Anspruch 1, dadurch gekennzeichnet, daß als Milzzellen die von BALB/cMäusen und als Myelomzellen die der Linie P3-X-63Ag8-6-5-3 verwendet werden.

3. Hybridzellinie EBI-1 gemäß den Ansprüchen 1 und 2, dadurch gekennzeichnet, daß sie permanent EBI-1 Antikörper synthetisiert.

4. Hybridzellinie EBI-2 gemäß den Ansprüchen 1 und 2, dadurch gekennzeichnet, daß sie permanent EBI-2-Antikörper synthetisiert.

5. Hybridzellinie EBI-3 gemäß den Ansprüchen 1 und 2, dadurch gekennzeichnet, daß sie permanent EBI-3-Antikörper synthetisiert.

6. Monoklonaler Antikörper, dadurch gekennzeichnet, daß er die Aktivität von Humaninterferon des Typs $\alpha$ ganz oder teilweise neutralisiert.

7. Monoklonaler Antikörper-EBI-1 des Typs IgG1 gemäß Anspruch 6, dadurch gekennzeichnet, daß
a) sein Molkulargewicht $\geq$ 150.000 Dalton beträgt,
b) die schwere Kette $\gamma 1$, die leichte Kette $\kappa$ ist,

c) er von Staphylococcus aureus Protein A gebunden wird,
d) er neutralisierende Wirkung auf die antivirale Aktivität von in E. coli produzierten Humaninterferon α2 (Arg) ausübt,
e) er neutralisierende Wirkung auf die antivirale Aktivität von Interferon-α, das ohne Induktor spontan in humanen lymphoblastoiden Zellen gebildet wird, ausübt,
f) er partiell neutralisierende Wirkung auf die antivirale Aktivität von natürlichen Gemischen verschiedener Subtypen des humanen α-Interferons ausübt,
g) er bei einer tausendfachen Verdünnung die antivirale Aktivität des Interferons-αC zu etwa 90 % neutralisiert,
h) er keine neutralisierende Wirkung gegen Humaninterferon-ß ausübt,
i) er keine neutralisierende Wirkung gegen Humaninterferon-γ ausübt und
j) er keine neutralisierende Wirkung gegen Mausinterferon ausübt.

8. Monoklonaler Antikörper EBI-2 des Typs IgG1 gemäß Anspruch 6, dadurch gekennzeichnet, daß
a) sein Molekulargewicht > 150.000 Dalton beträgt,
b) die schwere Kette γ1, die leichte Kette κ ist,
c) er von Staphylococcus aureus Protein A gebunden wird,
d) er neutralisierende Wirkung auf die antivirale Aktivität von in E. coli produzierten Humaninterferon α2(Arg) ausübt,
e) er neutralisierende Wirkung auf die antivirale Aktivität von Interferon-α, das ohne Induktor spontan in humanen lymphoblastoiden Zellen gebildet wird, ausübt,
f) er partielle neutralisierende Wirkung auf die antivirale Aktivität von natürlichen Gemischen verschiedener Subtypen des humanen α-Interferons ausübt und
g) er keine neutralisierende Wirkung gegen Interferon-αC ausübt.

9. Monoklonaler Antikörper EBI-3 des Typs IgG3 gemäß Anspruch 6, dadurch gekennzeichnet, daß
a) sein Molekulargewicht > 150.000 Dalton beträgt,

b) die schwere Kette γ3, die leichte Kette κ ist,
c) er von Staphylococcus aureus Protein A gebunden wird,
d) er neutralisierende Wirkung auf die antivirale Aktivität von in E. coli produzierten Humaninterferon α-2(Arg) ausübt,
e) er neutralisierende Wirkung auf die antivirale Aktivität von Interferon-α, das ohne Induktor spontan in humanen lymphoblastoiden Zellen gebildet wird, ausübt,
f) er partielle neutralisierende Wirkung auf die antivirale Aktivität von natürlichen Gemischen verschiedener Subtypen des humanen α-Interferons ausübt und
g) er bei einer tausendfachen Verdünnung die antivirale Aktivität des Interferons-αC zu etwa 40 % neutralisiert.

10. Verfahren zur Herstellung von neuen IgG-Antikörpern der Maus mit Anti-Humaninterferon α Spezifität gemäß Anspruch 6, dadurch gekennzeichnet, daß eine die IgG-Antikörper produzierende Hybridzellinie durch Zellfusion hergestellt und subkloniert wird und nach Wachstum der Zellen die IgG-Antikörper mit Anti-Interferon Spezifität isoliert werden.

11. Verfahren gemäß Anspruch 10, dadurch gekennzeichnet, daß zur Zellfusion Myelomzellen und Milzzellen von gegen Humaninterferon immunisierten Mäusen verwendet werden.

12. Verfahren gemäß Anspruch 11, dadurch gekennzeichnet, daß als Myelomzellen die Zellinie P3-X-63Ag8-6-5-3 verwendet wird.

13. Verfahren gemäß Anspruch 11, dadurch gekennzeichnet, daß als Milzzellen Zellen von gegen Humaninterferon immunisierten BALB/c-Mäusen verwendet werden.

14. Verfahren gemäß den Ansprüchen 11 und 13, dadurch gekennzeichnet, daß als Humaninterferon Humaninterferon α verwendet wird.

15. Verfahren zur Reinigung von Humaninterferon, dadurch gekennzeichnet, daß Humaninterferon des Typs α durch Chromatographie über eine IgG-Antikörper-Affinitätssäule gereinigt wird.

16. Verfahren gemäß Anspruch 15, dadurch gekennzeichnet, daß das über die IgG-Antikörper-Affinitätssäule gereinigte Humaninterferon anschließend über einen sauren Kationenaustauscher weitergereinigt wird.

17. Verfahren gemäß Anspruch 15, dadurch gekennzeichnet, daß als Antikörper, welcher kovalent am Träger gebunden ist, ein gemäß Anspruch 9 hergestellter IgG-Antikörper verwendet wird.

18. Verfahren gemäß Anspruch 16, dadurch gekennzeichnet, daß als Träger aktivierte Sepharose verwendet wird.

19. Verfahren gemäß Anspruch 16, dadurch gekennzeichnet, daß als Kationenaustauscher eine Mono-S-Chromatographiesäule verwendet wird.

20. Verfahren gemäß den Ansprüchen 15 bis 19, dadurch gekennzeichnet, daß beide Reinigungsschritte direkt nacheinander durchgeführt werden.

21. Verfahren zur Herstellung eines IgG-Antikörper-Affinitätsträgers, dadurch gekennzeichnet, daß ein gemäß Anspruch 10 hergestellter IgG-Antikörper kovalent an einen aktivierten Träger gebunden ist.

22. Verwendung der monoklonalen Antikörper gemäß den Ansprüchen 6 bis 9

a) zum Nachweis von Humaninterferon des Typs α, wobei sowohl die Interferon-α-Bindungen als auch die Interferon α neutralisierenden Eigenschaften der Antikörper eingesetzt werden,

b) zur diagnostischen Unterscheidung von Subtypen des Humaninterferon Typ α durch unterschiedliche Bindung und Neutralisation der antiviralen Aktivität der einzelnen Subtypen,

c) zur Reinigung von Humaninterferonen des Typs α, sowohl einzelner Subtypen desselben, als auch von Gemischen von α-Interferonsubtypen und deren Abtrennung von anderen Interferontypen.

23. Verwendung der monoklonalen Antikörper gemäß den Ansprüchen 6 bis 9 zum Nachweis, zur Diagnose oder zur Reinigung von Humaninterferonen des Typs α, wobei jeder der Antikörper für sich allein, in beliebiger Kombination miteinander, in Kombination mit anderen monovalenten Antikörpern miteinander, in Kombination mit anderen monovalenten Antikörpern oder in Kombination mit polyvalenten Antikörpergemischen eingesetzt werden kann.